(19) Europäisches Patentamt
European Patent Office
Office européen des brevets

(11) **EP 4 166 929 A1**

(12) **EUROPEAN PATENT APPLICATION**
published in accordance with Art. 153(4) EPC

(43) Date of publication:
**19.04.2023 Bulletin 2023/16**

(21) Application number: **21823112.4**

(22) Date of filing: **12.02.2021**

(51) International Patent Classification (IPC):
**G01N 21/27** $^{(2006.01)}$    **G01N 21/33** $^{(2006.01)}$
**G01N 31/00** $^{(2006.01)}$

(52) Cooperative Patent Classification (CPC):
**G01N 21/27; G01N 21/33; G01N 31/00**

(86) International application number:
**PCT/JP2021/005250**

(87) International publication number:
**WO 2021/250944 (16.12.2021 Gazette 2021/50)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.06.2020 JP 2020102236**

(71) Applicant: **SHIMADZU CORPORATION**
**Kyoto-shi, Kyoto 604-8511 (JP)**

(72) Inventor: **KITADA, Yoshio**
**Kyoto-shi, Kyoto 604-8511 (JP)**

(74) Representative: **Müller-Boré & Partner**
**Patentanwälte PartG mbB**
**Friedenheimer Brücke 21**
**80639 München (DE)**

(54) **WATER QUALITY ANALYZER AND WATER QUALITY ANALYSIS METHOD**

(57)    A correction calculation unit 102 performs calculation using a correction coefficient based on absorbance at each wavelength calculated by the absorbance calculation unit 101 to correct the absorbance. A turbidity component detection processing unit 103 supplies a turbidity standard solution to an oxidation reaction unit, and irradiates the turbidity standard solution after an oxidation reaction with measurement light from a light source 51, so as to cause a detector 52 to detect intensity at a plurality of wavelengths of the measurement light having passed through the turbidity standard solution. A correction coefficient update processing unit 104 causes the absorbance calculation unit 101 to calculate the absorbance at each wavelength based on the intensity at a plurality of wavelengths detected by the turbidity component detection processing unit 103, and updates a correction coefficient stored in a storage unit 200 based on the calculated absorbance at each wavelength.

FIG. 2

## Description

TECHNICAL FIELD

The present invention relates to a water quality analyzer and a water quality analysis method capable of measuring a total nitrogen concentration in sample water.

BACKGROUND ART

For example, a water quality analyzer such as a total nitrogen total phosphorus meter is provided with a reactor for oxidation reaction of a component in sample water. Sample water after oxidation reaction in the reactor is supplied to a measurement cell, and the sample water in the measurement cell is irradiated with measurement light from a light source. The measurement light having passed through the sample water in the measurement cell is detected by a detector, and the absorbance of a target component in the sample water is calculated based on detection intensity in the detector (see, for example, Patent Document 1 below).

PRIOR ART DOCUMENTS

PATENT DOCUMENTS

Patent Document 1: JP 2016-80441 A

SUMMARY OF THE INVENTION

PROBLEMS TO BE SOLVED BY THE INVENTION

In a case where a turbidity component is contained in sample water, not only absorption by a target component (for example, nitrogen oxide) but also absorption by the turbidity component occurs. For this reason, the absorbance of the target component cannot be accurately calculated. In view of the above, it is conceivable to correct the absorbance by performing calculation using a correction formula to remove the influence of absorption by a turbidity component.

For example, it is conceivable to perform calculation by calculating absorbance at each wavelength on the basis of detection intensity at each of a measurement wavelength corresponding to total nitrogen in sample water and a turbidity correction wavelength corresponding to a turbidity component, and substituting the absorbance as a variable into a correction formula. In this case, a constant obtained in advance by an experiment is used as a correction coefficient included in the correction formula.

Since the correction coefficient as described above is a constant obtained by an experiment, it is usually not necessary to change the correction coefficient frequently. However, as a result of intensive studies by the inventor from the viewpoint of setting a more appropriate correction coefficient, findings below have been obtained.

First, in a case where sample water containing a turbidity component is subjected to an oxidation reaction in a reactor, the form of the turbidity component may change. Specifically, it is considered that the absorbance depending on particle size also changes as particle size of a turbidity component changes with the oxidation reaction. In this case, if the correction coefficient is not changed according to a change amount of the particle size of the turbidity component, an error may occur in the corrected absorbance.

Further, a decomposition amount of an oxidant used in the oxidation reaction may be affected by a turbidity component. Specifically, in a case where a turbidity component is contained in sample water, it is conceivable that the turbidity component inhibits decomposition of the oxidant, and a residual amount of the oxidant after the oxidation reaction increases. Since an oxidant such as potassium peroxodisulfate has absorption at a measurement wavelength, in a case where a residual amount of this type of oxidant increases, the absorbance at the measurement wavelength may increase.

The present invention has been made in view of the above circumstances, and an object of the present invention is to provide a water quality analyzer and a water quality analysis method capable of appropriately correcting absorbance when measuring a total nitrogen concentration in sample water containing a turbidity component.

MEANS FOR SOLVING THE PROBLEMS

A first aspect of the present invention is a water quality analyzer capable of measuring a total nitrogen concentration in sample water containing a turbidity component, the water quality analyzer including an oxidation reaction unit, a light source, a detector, an absorbance calculation unit, a correction calculation unit, a storage unit, a turbidity component detection processing unit, and a correction coefficient update processing unit. The oxidation reaction unit causes a component in the sample water to undergo an oxidation reaction. The light source irradiates the sample water after the oxidation reaction in the oxidation reaction unit with measurement light. The detector detects intensity at a plurality of wavelengths of measurement light having passed through the sample water. The absorbance calculation unit calculates absorbance at each wavelength based on intensity of a plurality of wavelengths detected by the detector. The correction calculation unit corrects absorbance by performing calculation using a correction formula based on the absorbance at each wavelength calculated by the absorbance calculation unit. The storage unit stores a correction coefficient included in the correction formula. The turbidity component detection processing unit supplies a turbidity standard solution to the oxidation reaction unit, and irradiates the turbidity standard solution after the oxidation reaction with measurement light from the light source, so as to cause the detector to detect intensity at a plurality of the wavelengths of the measurement light having passed through the turbidity standard solution. The correction coefficient update processing unit causes the absorbance calculation unit to calculate absorbance at each wavelength based on the intensity at a plurality of the wavelengths detected by the turbidity component detection processing unit, and updates the correction coefficient stored in the storage unit based on the calculated absorbance at each wavelength.

A second aspect of the present invention is a water quality analysis method capable of measuring a total nitrogen concentration in sample water containing a turbidity component. The water quality analysis method includes the steps of oxidizing a component in the sample water, irradiating the sample water after the oxidation reaction with measurement light, detecting intensity at a plurality of wavelengths of measurement light having passed through the sample water, calculating absorbance at each wavelength based on the detected intensity at a plurality of the wavelengths, correcting absorbance by performing calculation using a correction formula based on the calculated absorbance at each wavelength, storing a correction coefficient included in the correction formula, causing a component in a turbidity standard solution to undergo an oxidation reaction, irradiating the turbidity standard solution after the oxidation reaction with measurement light, detecting intensity at a plurality of the wavelengths of measurement light that has passed through the turbidity standard solution, and calculating absorbance at each wavelength based on the detected intensity at a plurality of the wavelengths, and updating the stored correction coefficient based on the calculated absorbance at each wavelength.

EFFECTS OF THE INVENTION

According to the present invention, by supplying a turbidity standard solution to the oxidation reaction unit in the same manner as in analysis of sample water, a correction coefficient stored in the storage unit can be updated to an appropriate value based on detection intensity of measurement light having passed through the turbidity standard solution after an oxidation reaction, so that absorbance can be appropriately corrected when a total nitrogen concentration in the sample water containing a turbidity component is measured.

BRIEF DESCRIPTION OF THE DRAWINGS

FIG. 1 is a schematic diagram illustrating a configuration example of a water quality analyzer according to an embodiment of the present invention.
FIG. 2 is a block diagram illustrating an electrical configuration of the water quality analyzer of FIG. 1.
FIG. 3 is a diagram for explaining a specific mode of turbidity correction calibration.
FIG. 4 is a flowchart showing an example of a water quality analysis method using the water quality analyzer of FIG. 1.
FIG. 5 is a flowchart showing an example of the turbidity correction calibration.
FIG. 6 is a block diagram illustrating a variation of the water quality analyzer.

MODE FOR CARRYING OUT THE INVENTION

1. Overall configuration of water quality analyzer

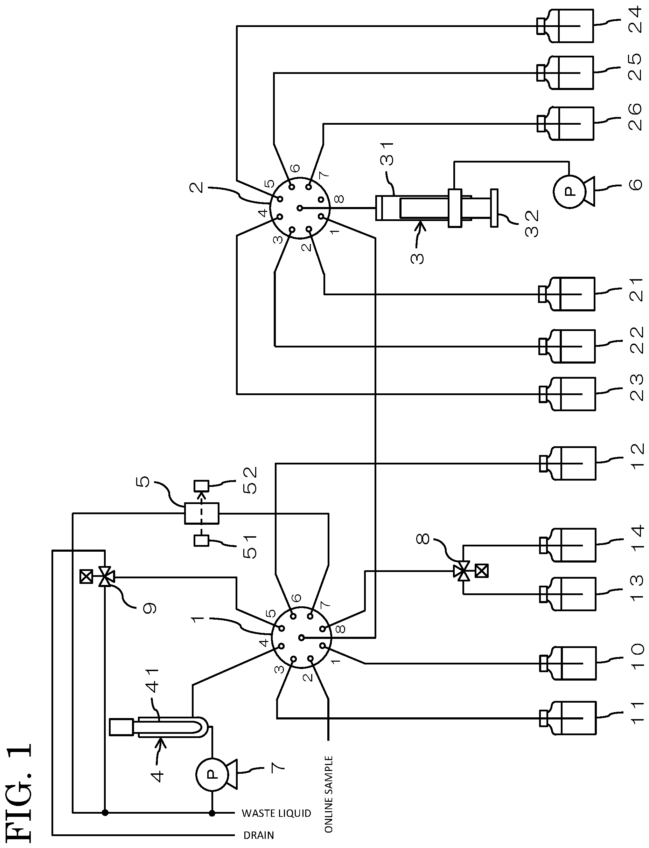

FIG. 1 is a schematic diagram illustrating a configuration example of a water quality analyzer according to an embodiment of the present invention. The water quality analyzer according to the present embodiment is a total nitrogen total phosphorus meter capable of measuring a total nitrogen concentration (TN concentration) and a total phosphorus concentration (TP concentration) of sample water, and only a configuration related to a flow path of liquid such as sample water is illustrated in FIG. 1.

Sample water is sewage, river water, factory wastewater, or the like, and contains various components such as a nitrogen compound and a phosphorus compound. A nitrogen compound in the sample water is present as, for example, a nitrate ion, a nitrite ion, an ammonium ion, and organic nitrogen. When a total nitrogen concentration of the sample water is measured, a nitrogen oxide (nitrate ion) is generated by oxidizing all nitrogen compounds in the sample water, and the concentration of the nitrogen oxides is measured.

Further, a phosphorus compound in the sample water is present as, for example, a phosphate ion, hydrolyzable phosphorus, and organic phosphorus. When a total phosphorus concentration in the sample water is measured, a phosphorus oxide (phosphate ion) is generated by oxidizing all phosphorus compounds in the sample water, and the concentration of the phosphorus oxide is measured.

The sample water contains a turbidity component in addition to a target component to be measured, such as a nitrogen oxide or a phosphorus oxide. The turbidity component is a component other than the target component, and is a component that generates turbidity in the sample water. When the turbidity component is contained in the sample water, when the sample water is irradiated with light, not only absorption by the target component but also absorption by the turbidity component occurs.

The water quality analyzer according to the present embodiment includes, for example, a first multi-port valve 1, a second multi-port valve 2, a syringe 3, a reactor (reactor) 4, a measurement cell 5, a stirring pump 6, a discharge pump 7, a first switching valve 8, a second switching valve 9, and the like. These units are connected to each other via a pipe.

The first multi-port valve 1 and the second multi-port valve 2 include, for example, an eight-port valve, and include one common port and eight ports (first to eighth ports) selectively communicable with the common port. In FIG. 1, ports of the first multi-port valve 1 and the second multi-port valve 2 are indicated by numbers "1" to "8" in association with the first to eighth ports, respectively.

The common port of the first multi-port valve 1 is connected to the first port of the second multi-port valve 2. The common port of the second multi-port valve 2 is connected to the syringe 3. The syringe 3 is provided with, for example, a cylindrical body 31 and a plunger 32, and suction operation to the syringe 3 and discharge operation from the syringe 3 can be performed as the plunger 32 inserted into the cylindrical body 31 is displaced.

A turbidity standard solution storage portion 10 storing a turbidity standard solution is connected to the first port of the first multi-port valve 1. During operation of the water quality analyzer, the turbidity standard solution in the turbidity standard solution storage portion 10 may be stirred by a stirring device (not illustrated). When suction operation by the syringe 3 is performed in a state in which the first port of the first multi-port valve 1 communicates with the common port and the first port of the second multi-port valve 2 communicates with the common port, the turbidity standard solution can be supplied into the syringe 3. The turbidity standard solution is a solution containing a component to be a reference in determining turbidity. As the turbidity standard solution, for example, a kaolin solution can be exemplified, but the turbidity standard solution is not limited to this, and any other turbidity standard solution can be stored in the turbidity standard solution storage portion 10.

Sample water is supplied online from a sample water supply source such as a drainage facility to the second port of the first multi-port valve 1 via piping. Therefore, when suction operation by the syringe 3 is performed in a state in which the second port of the first multi-port valve 1 communicates with the common port and the first port of the second multi-port valve 2 communicates with the common port, the sample water can be supplied into the syringe 3 online. At this time, a sample supplied online is supplied to the second port after predetermined preprocessing is performed by, for example, a preprocessing device (not illustrated). However, the present invention is not limited to the online configuration, and sample water collected in advance and set in the water quality analyzer may be configured to be supplied offline.

Reagent storage portions 21 to 26 in which different reagents are stored are connected to the second to seventh ports of the second multi-port valve 2. By causing any of these ports to communicate with the common port and performing suction operation by the syringe 3, a reagent can be supplied into the syringe 3 and mixed with sample water. Examples of the reagents stored in the reagent storage portions 21 to 26 include sulfuric acid, molybdic acid, ascorbic acid, sodium hydroxide, potassium peroxodisulfate, and hydrochloric acid, but are not limited to these, and any other reagent can be stored in the reagent storage portions 21 to 26.

A dilution water storage portion 12 is connected to the sixth port of the first multi-port valve 1. Dilution water used for diluting sample water, cleaning the reactor 4 or the measurement cell 5, or the like is stored in the dilution water storage portion 12. As the dilution water, for example, pure water can be used. When suction operation by the syringe 3 is performed in a state in which the sixth port of the first multi-port valve 1 communicates with the common port and the first port of the second multi-port valve 2 communicates with the common port, the dilution water can be supplied into the syringe 3 and mixed with sample water.

As described above, by appropriately switching the first multi-port valve 1 and the second multi-port valve 2 and performing suction operation by the syringe 3, a mixed solution of sample water, a reagent, and dilution water can be generated in the syringe 3. The mixed solution in the syringe 3 is stirred by driving of the stirring pump 6. The reactor 4 is connected to the fourth port of the first multi-port valve 1, and when discharge operation by the syringe 3 is performed in a state where the fourth port communicates with the common port and the first port of the second multi-port valve 2 communicates with the common port, the mixed solution in the syringe 3 can be supplied to the reactor 4.

In the reactor 4, liquid inside is irradiated with an ultraviolet ray from a light source 41. In a case where sample water, a reagent, and dilution water are supplied to the reactor 4 as a mixed solution, various components such as a nitrogen compound and a phosphorus compound in the sample water can be oxidized by irradiating the mixed solution with an ultraviolet ray from the light source 41. That is, the reactor 4 constitutes an oxidation reaction unit that causes a component in the sample water to undergo an oxidation reaction. At this time, the reagent in the mixed solution can function as an oxidant. As the oxidant, for example, potassium peroxodisulfate may be used. However, an oxidation reaction in the reactor 4 is not limited to the configuration in which the oxidation reaction is performed by irradiation with an ultraviolet ray, and the oxidation reaction may be performed in another mode such as control of pressure and temperature.

On the other hand, when discharge operation by the syringe 3 is performed in a state where the fourth port and the common port communicate with each other and the first port and the common port of the second multi-port valve 2 communicate with each other after a turbidity standard solution is supplied into the syringe 3, the turbidity standard solution in the syringe 3 can also be supplied to the reactor 4. In this case, a reagent (for example, potassium peroxodisulfate) as an oxidant may be sucked into the syringe 3 to mix the oxidant with the turbidity standard solution. Further, the turbidity standard solution may be brought into an alkaline atmosphere by suctioning of a reagent such as sodium hydroxide into the syringe 3. In a case where the turbidity standard solution is supplied to the reactor 4, the turbidity standard solution is irradiated with an ultraviolet ray from the light source 41.

As the light source 41, for example, a low-pressure mercury lamp can be used, but the light source 41 is not limited to this. Another type of the light source 41 such as an excimer laser, a deuterium lamp, a xenon lamp, or a Hg-Zn-Pb lamp may be used. Liquid in the reactor 4 is heated by, for example, a heater (not illustrated). At this time, the temperature of the liquid in the reactor 4 is controlled to be a preset temperature based on a detection signal from a temperature sensor (not illustrated) that detects the temperature of the liquid.

Liquid after an oxidation reaction in the reactor 4 is supplied into the syringe 3 by suction operation by the syringe 3. The measurement cell 5 is connected to the seventh port of the first multi-port valve 1, and when discharge operation by the syringe 3 is performed in a state where the seventh port and the common port communicate with each other and the first port of the second multi-port valve 2 and the common port communicate with each other, the liquid after an oxidation reaction in the syringe 3 can be supplied to the measurement cell 5.

Liquid (mixed solution or turbidity standard liquid) after an oxidation reaction in the measurement cell 5 is irradiated with measurement light from a light source 51. As the light source 51, for example, a xenon lamp that emits white light can be used, but the light source 51 is not limited to this, and another type of the light source 51 such as a deuterium lamp or a tungsten lamp may be used. Note that, in a case where the liquid after an oxidation reaction (mixed solution or turbidity standard solution) sucked into the syringe 3 is in an alkaline atmosphere, a reagent such as hydrochloric acid may be sucked into the syringe 3 to cause the liquid after an oxidation reaction to be in an acidic atmosphere, and then the liquid may be supplied to the measurement cell 5.

Measurement light transmitted through the measurement cell 5 is detected by a detector 52 such as a photodiode. In a case where the mixed solution (mixed solution of sample water, a reagent and dilution water) after an oxidation reaction is supplied into the measurement cell 5, the measurement light passing through the mixed solution is detected by the detector 52 so that the total nitrogen concentration or the total phosphorus concentration in sample water can be measured based on a detection signal of the detection. The liquid in the measurement cell 5 is heated by, for example, a heater (not illustrated). At this time, the temperature of the liquid in the measurement cell 5 is controlled to be a preset temperature based on a detection signal from a temperature sensor (not illustrated) that detects the temperature of the liquid.

Note that dilution water can also be individually flown into the reactor 4 and the measurement cell 5. That is, the inside of the reactor 4 can be cleaned by individually sucking dilution water into the syringe 3 and allowing the dilution water to flow into the reactor 4 from the syringe 3. Further, the inside of the measurement cell 5 can be cleaned by individually sucking dilution water into the syringe 3 and allowing the dilution water to flow from the syringe 3 into the measurement cell 5.

At the time of performing zero calibration, it is possible to supply dilution water from the dilution water storage portion 12 into the syringe 3 by suction operation by the syringe 3 in a state where the sixth port of the first multi-port valve 1 and the common port communicate with each other and the first port of the second multi-port valve 2 and the common port communicate with each other. After the above, the seventh port of the first multi-port valve 1 and the common port are made to communicate with each other, and discharge operation by the syringe 3 is performed, so that the dilution water can be supplied into the measurement cell 5 to perform zero calibration.

A span solution storage portion 11 is connected to the third port of the first multi-port valve 1. The span solution storage portion 11 stores a span solution used when span calibration is performed. When suction operation by the syringe 3 is performed in a state in which the third port of the first multi-port valve 1 communicates with the common port and the first port of the second multi-port valve 2 communicates with the common port, the span solution can be supplied from the span solution storage portion 11 into the syringe 3. After the above, the seventh port of the first multi-port valve 1 and the common port are made to communicate with each other, and discharge operation by the syringe 3 is performed, so that the span solution can be supplied into the measurement cell 5 to perform span calibration.

Two standard sample storage portions 13 and 14 are connected to the eighth port of the first multi-port valve 1 via the first switching valve 8. A standard sample is stored in each of the standard sample storage portions 13 and 14, a standard sample for measuring the total nitrogen concentration is stored in the standard sample storage portion 13 on the one hand, and a standard sample for measuring the total phosphorus concentration is stored in the standard sample storage portion 14 on the other. The first switching valve 8 can selectively allow one of two of the standard sample storage portions 13 and 14 to communicate with the eighth port by switching a flow path.

When suction operation by the syringe 3 is performed in a state in which the eighth port of the first multi-port valve 1 communicates with the common port and the first port of the second multi-port valve 2 communicates with the common port, a standard sample can be supplied from one of two of the standard sample storage portions 13 and 14 into the syringe 3. After the above, the standard sample can be supplied into the measurement cell 5 by causing the seventh port of the first multi-port valve 1 and the common port to communicate with each other and performing discharge operation by the syringe 3.

The liquid in the measurement cell 5 is discharged to the outside of the device. Further, the liquid in the reactor 4 is also discharged to the outside of the device by driving of the discharge pump 7. The fifth port of the first multi-port valve 1 communicates with a waste liquid destination and a drain destination via the second switching valve 9. The second switching valve 9 can selectively guide the liquid in the device to either the waste liquid destination or the drain destination by switching a flow path.

2. Electrical configuration of water quality analyzer

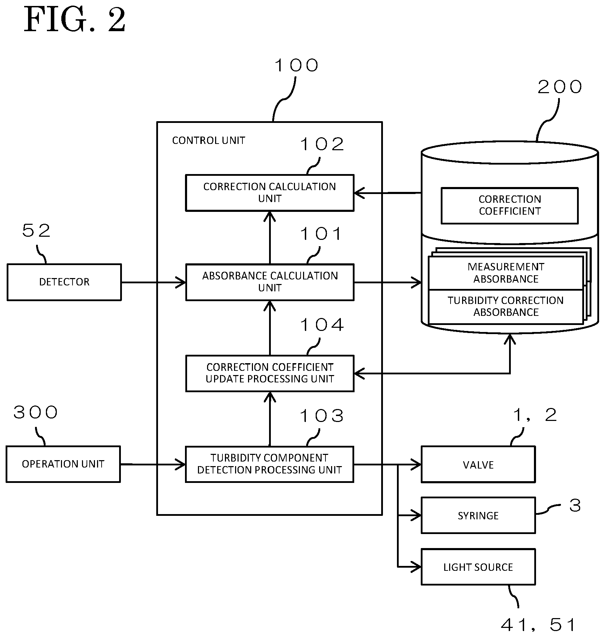

FIG. 2 is a block diagram illustrating an electrical configuration of the water quality analyzer of FIG. 1. Operation of the water quality analyzer is controlled by a control unit 100 including, for example, a central processing unit (CPU). In addition to the above-described units, a storage unit 200, an operation unit 300, and the like are electrically connected to the control unit 100.

When the CPU executes a program, the control unit 100 functions as an absorbance calculation unit 101, a correction calculation unit 102, a turbidity component detection processing unit 103, a correction coefficient update processing unit 104, and the like. The storage unit 200 includes, for example, a random access memory (RAM), a read only memory (ROM), or a hard disk, and stores data necessary for operation of the water quality analyzer. The operation unit 300 includes, for example, a touch panel, a keyboard, or a mouse, and is operated by the user.

The absorbance calculation unit 101 calculates the absorbance based on a detection signal from the detector 52. A half mirror, an optical filter (none of which are illustrated), and the like are provided between the light source 51 and the detector 52. Measurement light (white light) emitted from the light source 51 is divided into a plurality of rays of light by the half mirror before or after passing through the measurement cell 5, and each ray of light enters the detector 52 after passing through different optical filters.

Each optical filter transmits only light of a specific wavelength. In the present embodiment, an optical filter that transmits a measurement wavelength (for example, 220 nm) and an optical filter that transmits a turbidity correction wavelength (for example, 275 nm) are provided. The measurement light emitted from the light source 51 is divided into two rays of light by the half mirror and passes through different optical filters, so that light of each of the measurement wavelength and the turbidity correction wavelength is incident on the detector 52.

By the above, the detector 52 can detect the intensity at two wavelengths (measurement wavelength and turbidity correction wavelength) of the measurement light having passed through sample water in the measurement cell 5. However, it is also possible to employ a configuration in which the measurement light is divided into three or more rays of light by providing a plurality of half mirrors, and these rays of light pass through three or more optical filters and enter the detector 52. That is, the detector 52 only needs to be able to detect intensities at a plurality of wavelengths.

The absorbance calculation unit 101 calculates absorbance at each wavelength based on intensity of a plurality of (for example, two) wavelengths detected by the detector 52. In the present embodiment, the measurement wavelength is a wavelength corresponding to the total nitrogen in sample water, and nitrogen oxide (target component) in the sample water has absorption at the measurement wavelength. On the other hand, the turbidity correction wavelength is a wavelength corresponding to a turbidity component in sample water, and a turbidity component in the sample water has absorption at the measurement wavelength and the turbidity correction wavelength. The turbidity correction wavelength is longer than the measurement wavelength.

The absorbance at each wavelength calculated by the absorbance calculation unit 101 is stored in the storage unit 200. At this time, the absorbance at the measurement wavelength is stored in the storage unit 200 as measurement absorbance. On the other hand, the absorbance at the turbidity correction wavelength is stored in the storage unit 200 as turbidity correction absorbance.

The correction calculation unit 102 performs calculation using Correction formulas (1) and (2) below based on the absorbance at each wavelength (measurement wavelength and turbidity correction wavelength) to correct the absorbance.

$$A = B \cdot C \dots \quad (1)$$

$$C = kD + m \dots \quad (2)$$

Here, A is the absorbance after correction. B is the absorbance at the measurement wavelength (measurement absorbance). C is the absorbance at the turbidity correction wavelength (turbidity correction absorbance) corrected and converted into the absorbance corresponding to the measurement wavelength. D is the absorbance at the turbidity correction wavelength (turbidity correction absorbance). The term k is a proportional term of the correction coefficient. The term m is a constant term of the correction coefficient.

The correction calculation unit 102 can remove the influence of absorption by a turbidity component and accurately calculate the absorbance of a target component by performing calculation of substituting the measurement absorbance B and the turbidity correction absorbance D as variables into Correction formulas (1) and (2). The correction coefficients k and m are stored in the storage unit 200.

In the present embodiment, a series of operation (turbidity correction calibration) for updating the correction coefficients k and m stored in the storage unit 200 can be performed so that the correction coefficients k and m can be updated to appropriate values. Specifically, the turbidity component detection processing unit 103 supplies the turbidity standard solution from the turbidity standard solution storage portion 10 to the reactor 4, irradiates the turbidity standard solution with an ultraviolet ray from the light source 41 to perform an oxidation reaction on the turbidity standard solution, supplies the turbidity standard solution after the oxidation reaction to the measurement cell 5, and irradiates the turbidity standard solution with measurement light from the light source 51. By the above, intensity at the measurement wavelength and the turbidity correction wavelength of the measurement light having passed through the turbidity standard solution after the oxidation reaction is detected by the detector 52.

As described above, in the turbidity correction calibration, operation of the first multi-port valve 1, the second multi-port valve 2, the syringe 3, the light sources 41 and 51, and the like are appropriately controlled by the turbidity component detection processing unit 103. The turbidity correction calibration is started based on, for example, operation of the operation unit 300 by the user. In this case, an operation screen may be displayed on a display unit (not illustrated), and the user may perform input on the operation screen by operating the operation unit 300.

Further, in the turbidity correction calibration, the correction coefficient update processing unit 104 causes the absorbance calculation unit 101 to calculate the measurement absorbance and the turbidity correction absorbance based on intensity at the measurement wavelength and the turbidity correction wavelength detected by the turbidity component detection processing unit 103. Then, the correction coefficient update processing unit 104 updates the correction coefficients k and m stored in the storage unit 200 based on the calculated measurement absorbance and turbidity correction absorbance.

3. Turbidity correction calibration

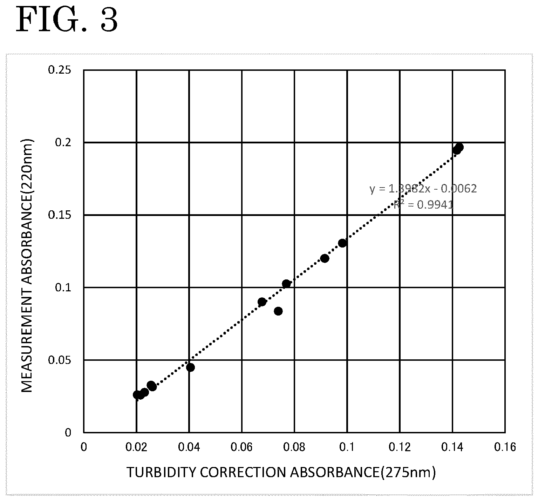

FIG. 3 is a diagram for describing a specific mode of the turbidity correction calibration. In this example, a case in which a plurality of sets of absorbance data are acquired with the measurement absorbance and the turbidity correction absorbance calculated by one time of measurement using the turbidity standard solution as one set of absorbance data, and then the correction coefficients k and m are updated based on the absorbance data will be described.

Here, "one time of measurement using the turbidity standard solution" means operation of detecting the measurement absorbance and the turbidity correction absorbance based on intensity at the measurement wavelength and the turbidity correction wavelength obtained by supplying the turbidity standard solution to the reactor 4 and irradiating the turbidity standard solution after an oxidation reaction with measurement light from the light source 51. In this example, a plurality of sets of absorbance data are acquired by performing measurement using each turbidity standard solution once or a plurality of times using a plurality of types of the turbidity standard solution having different concentrations. The turbidity standard solution having different concentrations can be generated by supplying dilution water in the dilution water storage portion 12 into the syringe 3 to dilute the turbidity standard solution.

In FIG. 3, a plurality of sets of acquired absorbance data are plotted on a graph in which the vertical axis represents the measurement absorbance (220 nm) and the horizontal axis represents the turbidity correction absorbance (275 nm). The measurement absorbance plotted on the graph may be a value obtained by subtracting the absorbance at a zero point (zero absorbance) obtained by zero calibration from the measurement absorbance calculated by measurement using the turbidity standard solution.

As calculation using the least squares method is performed on a plurality of points plotted on the graph in this manner, a linear function as illustrated by a straight line in FIG. 3 is obtained. This linear function represents a relationship between the measurement absorbance and the turbidity correction absorbance, and is expressed by Correction formula (2) described above in a case where a value on the vertical axis is C and a value on the horizontal axis is D. Therefore, the proportional term k and the constant term m of the correction coefficient in Correction formula (2) can be obtained based on the linear function obtained by the least squares method.

The correction coefficient update processing unit 104 updates the correction coefficients k and m by overwriting the correction coefficients k and m stored in the storage unit 200 with the correction coefficients k and m newly obtained. However, other calculation may be performed in addition to the calculation using the least squares method or instead of the calculation using the least squares method. In this case, the calculation may be performed such that the value of the constant term m becomes "0". Further, a plurality of times of measurement may be performed using one kind of the turbidity standard solution, or only one time of measurement may be performed. In a case where measurement is performed only once, a straight line connecting a point of an obtained set of absorbance data and the origin may be obtained as a linear function.

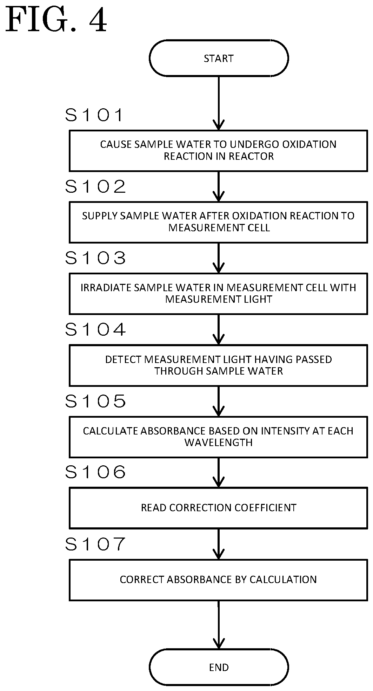

FIG. 4 is a flowchart showing an example of a water quality analysis method using the water quality analyzer of FIG. 1. When water quality of sample water is analyzed, the sample water (mixed solution) is supplied to the reactor 4 to cause a component in the sample water to undergo an oxidation reaction in the reactor 4 (step S101). Then, the sample water after the oxidation reaction is supplied to the measurement cell 5 (step S102), and the sample water in the measurement cell 5 is irradiated with measurement light from the light source 51 (step S103). By the above, the measurement light having passed through the sample water is detected by the detector 52 (step S104).

The detector 52 detects intensity of a plurality of (for example, two) wavelengths. The absorbance calculation unit 101 calculates the absorbance at each wavelength based on the intensity at each wavelength (step S105). After the above, the correction calculation unit 102 reads the correction coefficient from the storage unit 200 (step S 106), and performs calculation using Correction formulas (1) and (2) based on the calculated absorbance at each wavelength, so that the absorbance is corrected (step S107). The correction coefficient included in Correction formulas (1) and (2) are stored in the storage unit 200 in advance.

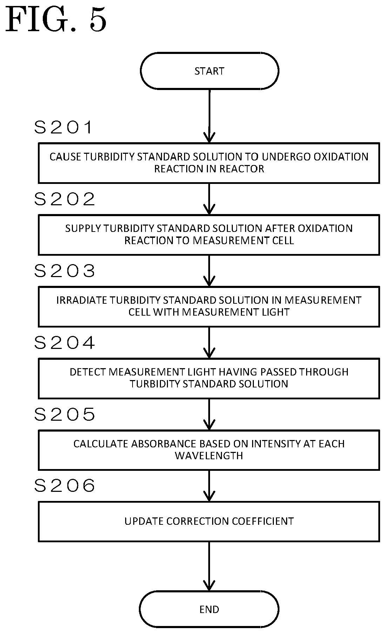

FIG. 5 is a flowchart showing an example of the turbidity correction calibration. When the turbidity correction calibration is performed, the turbidity standard solution is supplied to the reactor 4 to cause a component in the turbidity standard solution to undergo an oxidation reaction in the reactor 4 (step S201). Then, the turbidity standard solution after the oxidation reaction is supplied to the measurement cell 5 (step S202), and the turbidity standard solution in the measurement cell 5 is irradiated with measurement light from the light source 51 (step S203). By the above, the measurement light that has passed through the turbidity standard solution is detected by the detector 52 (step S204).

The detector 52 detects intensity of a plurality of (for example, two) wavelengths. The absorbance calculation unit 101 calculates the absorbance at each wavelength based on the intensity at each wavelength (step S205). After the above, the correction coefficient update processing unit 104 updates the correction coefficient stored in the storage unit 200 based on the calculated absorbance at each wavelength (step S206).

However, the configuration may be such that at least one of the steps illustrated in FIGS. 4 and 5 is manually performed by an operator.

4. Variation of water quality analyzer

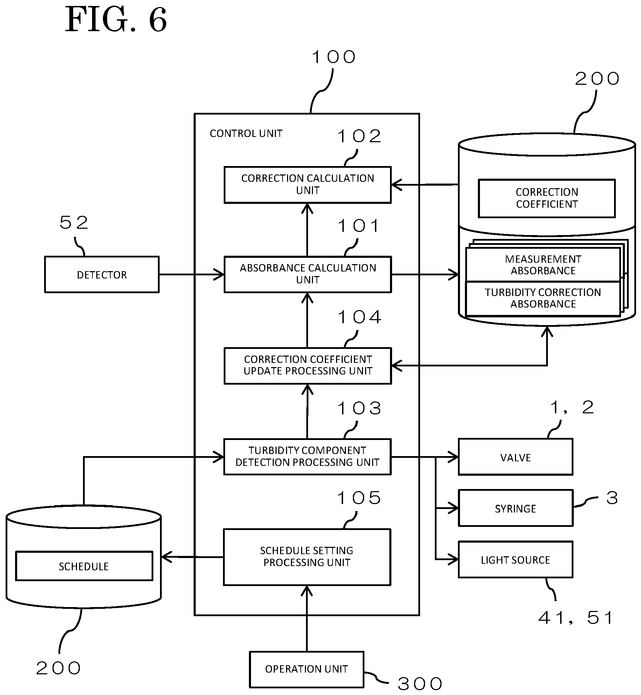

FIG. 6 is a block diagram illustrating a variation of the water quality analyzer. In the above embodiment, the configuration in which the turbidity correction calibration is started based on operation of the operation unit 300 by the user is described. On the other hand, in the variation of FIG. 6, the turbidity correction calibration is automatically started according to a preset schedule. In this variation, only the configuration for automatically starting the turbidity correction calibration is different from that of the above embodiment, and the other configurations are similar to those of the above embodiment. Therefore, the similar configurations are denoted by the same reference numerals in the diagram, and detailed description of them is omitted.

In this variation, the control unit 100 functions as a schedule setting processing unit 105 when the CPU executes a program. The schedule setting processing unit 105 sets the start date and time of the turbidity correction calibration as a schedule on the basis of operation of the operation unit 300 by the user, and stores the set schedule in the storage unit 200. In this case, an operation screen for the user to set the schedule may be displayed on a display unit (not illustrated).

The turbidity component detection processing unit 103 automatically starts supply of the turbidity standard solution to the reactor 4 based on the schedule stored in the storage unit 200, so as to execute the turbidity correction calibration at a frequency corresponding to the schedule. However, the schedule of the turbidity correction calibration is not limited to the configuration in which the user operates the operation unit 300, and may be automatically set based on operation time of the water quality analyzer or the like.

5. Other variations

In the above embodiment, the case where the turbidity correction wavelength is 275 nm is described, but other wavelengths may be used. For example, the turbidity correction wavelength may be a wavelength corresponding to the total phosphorus in sample water. The "wavelength corresponding to the total phosphorus in sample water" is a wavelength at which phosphorus oxide (target component) in the sample water has absorption, and is, for example, 880 nm. In this case, since an optical filter that transmits the measurement wavelength (for example, 880 nm) when the total phosphorus concentration is measured can be used as an optical filter that transmits the turbidity correction wavelength, it is not necessary to separately provide an optical filter for the turbidity correction calibration.

Further, in the above embodiment, the case where the water quality analyzer is a total nitrogen total phosphorus meter is described, but the present invention is also applicable to a total nitrogen meter. That is, the present invention can be applied to a total nitrogen meter that can measure the total nitrogen concentration but cannot measure the total phosphorus concentration.

The water quality analyzer is not limited to the configuration including two of the multi-port valves 1 and 2, and may have a configuration including only one multi-port valve or a configuration including three or more multi-port valves. Further, the type and number of valves, pipes, and the like are optional, and are not limited to those in the configuration as in the above embodiment. For example, it is also possible to employ a configuration in which the reactor 4 also functions as the measurement cell 5.

6. Aspect

It is understood by those skilled in the art that a plurality of the exemplary embodiments described above are specific examples of an aspect below.

(Clause 1) A water quality analyzer according to an aspect is a water quality analyzer capable of measuring a total nitrogen concentration in sample water containing a turbidity component. The water quality analyzer may include:

an oxidation reaction unit that causes a component in the sample water to undergo an oxidation reaction;
a light source that irradiates the sample water after an oxidation reaction in the oxidation reaction unit with measurement light;
a detector that detects intensity at a plurality of wavelengths of measurement light having passed through the sample water;
an absorbance calculation unit that calculates absorbance at each wavelength based on intensity of a plurality of wavelengths detected by the detector;
a correction calculation unit that corrects absorbance by performing calculation using a correction formula based on the absorbance at each wavelength calculated by the absorbance calculation unit;
a storage unit that stores a correction coefficient included in the correction formula;
a turbidity component detection processing unit that supplies a turbidity standard solution to the oxidation reaction unit and irradiates the turbidity standard solution after an oxidation reaction with measurement light from the light source to cause the detector to detect intensity at a plurality of the wavelengths of measurement light having passed through the turbidity standard solution; and
a correction coefficient update processing unit that causes the absorbance calculation unit to calculate absorbance at each wavelength based on the intensity at a plurality of the wavelengths detected by the turbidity component detection processing unit, and updates the correction coefficient stored in the storage unit based on the calculated absorbance at each wavelength.

According to the water quality analyzer described in Clause 1, by supplying a turbidity standard solution to the oxidation reaction unit in the same manner as in analysis of sample water, a correction coefficient stored in the storage unit can be updated to an appropriate value based on detection intensity of measurement light having passed through the turbidity standard solution after an oxidation reaction, so that absorbance can be appropriately corrected when a total nitrogen concentration in the sample water containing a turbidity component is measured.

(Clause 2) In the water quality analyzer according to Clause 1,

the plurality of the wavelengths may include a measurement wavelength corresponding to total nitrogen in the sample water and a turbidity correction wavelength longer than the measurement wavelength.

According to the water quality analyzer described in Clause 2, intensity at two wavelengths of the measurement wavelength corresponding to the total nitrogen in the sample water and the turbidity correction wavelength longer than the measurement wavelength is detected by the detector, and a correction coefficient stored in the storage unit can be updated to an appropriate value based on the detected intensity.

(Clause 3) In the water quality analyzer according to Clause 2,

the correction coefficient may include a proportional term multiplied by absorbance at the turbidity correction wavelength.

According to the water quality analyzer described in Clause 3, since the proportional term multiplied by the absorbance at the turbidity correction wavelength can be updated to an appropriate value, the absorbance can be appropriately corrected when the total nitrogen concentration in the sample water containing a turbidity component is measured.

(Clause 4) In the water quality analyzer according to Clause 2 or 3,

the turbidity correction wavelength may be a wavelength corresponding to total phosphorus in the sample water.

According to the water quality analyzer described in Clause 4, in the water quality analyzer capable of measuring not only the total nitrogen concentration but also the total phosphorus concentration, the measurement wavelength at the time of measuring the total phosphorus concentration can be used as the turbidity correction wavelength.

(Clause 5) In the water quality analyzer according to any one of Clauses 1 to 4,

the turbidity component detection processing unit may automatically start supply of a turbidity standard solution to the oxidation reaction unit.

According to the water quality analyzer described in Clause 5, supply of the turbidity standard solution to the oxidation reaction unit can be automatically started, and the correction coefficient stored in the storage unit can be automatically updated.

(Clause 6) In the water quality analyzer according to any one of Clauses 1 to 5,

the oxidation reaction unit may perform an oxidation reaction using potassium peroxodisulfate as an oxidant.

According to the water quality analyzer described in Clause 6, the absorbance can be appropriately corrected in the water quality analyzer capable of measurement using potassium peroxodisulfate as an oxidant.

(Clause 7) In the water quality analyzer according to any one of Clauses 1 to 6,

in the oxidation reaction unit, a component in the sample water may undergo an oxidation reaction as the sample water is irradiated with an ultraviolet ray.

According to the water quality analyzer described in Clause 7, the absorbance can be appropriately corrected in the water quality analyzer capable of performing measurement using ultraviolet absorptiometry or the like.

(Clause 8) A water quality analysis method according to an aspect is a water quality analysis method capable of measuring a total nitrogen concentration in sample water containing a turbidity component. The water quality analysis method includes the steps of

oxidizing a component in the sample water;
irradiating the sample water after the oxidation reaction with measurement light;
detecting intensity at a plurality of wavelengths of measurement light having passed through the sample water;
calculating absorbance at each wavelength based on the detected intensity at a plurality of the wavelengths;
correcting absorbance by performing calculation using a correction formula based on the calculated absorbance at each wavelength;
storing a correction coefficient included in the correction formula;
causing a component in a turbidity standard solution to undergo an oxidation reaction;
irradiating the turbidity standard solution after the oxidation reaction with measurement light;
detecting intensity at a plurality of the wavelengths of measurement light that has passed through the turbidity standard solution; and
calculating absorbance at each wavelength based on the detected intensity at a plurality of the wavelengths, and updating the stored correction coefficient based on the calculated absorbance at each wavelength.

According to the water quality analysis method described in Clause 8, by causing a turbidity standard solution to undergo an oxidation reaction in the same manner as in analysis of sample water, a correction coefficient included in the correction coefficient can be updated to an appropriate value based on detection intensity of measurement light having passed through the turbidity standard solution after the oxidation reaction, so that absorbance can be appropriately corrected when a total nitrogen concentration in the sample water containing a turbidity component is measured.

DESCRIPTION OF REFERENCE SIGNS

| 4 | reactor |
| 5 | measurement cell |
| 41 | light source |
| 51 | light source |
| 52 | detector |
| 100 | control unit |
| 101 | absorbance calculation unit |
| 102 | correction calculation unit |
| 103 | turbidity component detection processing unit |
| 104 | correction coefficient update processing unit |
| 105 | schedule setting processing unit |
| 200 | storage unit |
| 300 | operation unit |

**Claims**

1. A water quality analyzer capable of measuring a total nitrogen concentration in sample water containing a turbidity component, the water quality analyzer comprising:

an oxidation reaction unit that causes a component in the sample water to undergo an oxidation reaction;
a light source that irradiates the sample water after an oxidation reaction in the oxidation reaction unit with measurement light;
a detector that detects intensity at a plurality of wavelengths of measurement light having passed through the sample water;
an absorbance calculation unit that calculates absorbance at each wavelength based on intensity of a plurality of wavelengths detected by the detector;
a correction calculation unit that corrects absorbance by performing calculation using a correction formula based on the absorbance at each wavelength calculated by the absorbance calculation unit;
a storage unit that stores a correction coefficient included in the correction formula;
a turbidity component detection processing unit that supplies a turbidity standard solution to the oxidation reaction unit and irradiates the turbidity standard solution after an oxidation reaction with measurement light from the light source to cause the detector to detect intensity at the plurality of wavelengths of measurement light having passed through the turbidity standard solution; and
a correction coefficient update processing unit that causes the absorbance calculation unit to calculate absorbance at each wavelength based on the intensity at the plurality of wavelengths detected by the turbidity component detection processing unit, and updates the correction coefficient stored in the storage unit based on the calculated absorbance at each wavelength.

2. The water quality analyzer according to claim 1, wherein

the plurality of wavelengths include a measurement wavelength corresponding to total nitrogen in the sample water and a turbidity correction wavelength longer than the measurement wavelength.

3. The water quality analyzer according to claim 2, wherein

the correction coefficient includes a proportional term multiplied by absorbance at the turbidity correction wavelength.

4. The water quality analyzer according to claim 2 or 3, wherein

the turbidity correction wavelength is a wavelength corresponding to total phosphorus in the sample water.

5. The water quality analyzer according to any one of claims 1 to 4, wherein

the turbidity component detection processing unit automatically starts supply of a turbidity standard solution to the oxidation reaction unit.

6. The water quality analyzer according to any one of claims 1 to 5, wherein

the oxidation reaction unit performs an oxidation reaction using potassium peroxodisulfate as an oxidant.

7. The water quality analyzer according to any one of claims 1 to 6, wherein

in the oxidation reaction unit, a component in the sample water undergoes an oxidation reaction as the sample water is irradiated with an ultraviolet ray.

8. A water quality analysis method capable of measuring a total nitrogen concentration in sample water containing a turbidity component, the water quality analysis method comprising the steps of

oxidizing a component in the sample water;
irradiating the sample water after the oxidation reaction with measurement light;
detecting intensity at a plurality of wavelengths of measurement light having passed through the sample water;
calculating absorbance at each wavelength based on the detected intensity at the plurality of wavelengths;
correcting absorbance by performing calculation using a correction formula based on the calculated absorbance at each wavelength;
storing a correction coefficient included in the correction formula;
causing a component in a turbidity standard solution to undergo an oxidation reaction;
irradiating the turbidity standard solution after the oxidation reaction with measurement light;

detecting intensity at the plurality of wavelengths of measurement light that has passed through the turbidity standard solution; and
calculating absorbance at each wavelength based on the detected intensity at the plurality of wavelengths, and updating the stored correction coefficient based on the calculated absorbance at each wavelength.

# FIG. 1

FIG. 2

# FIG. 3

FIG. 4

```
                    ┌──────────────────┐
                    │      START       │
                    └────────┬─────────┘
                             │
  S101                       ▼
          ┌──────────────────────────────────────────┐
          │  CAUSE SAMPLE WATER TO UNDERGO OXIDATION  │
          │            REACTION IN REACTOR            │
          └──────────────────┬───────────────────────┘
  S102                       ▼
          ┌──────────────────────────────────────────┐
          │  SUPPLY SAMPLE WATER AFTER OXIDATION      │
          │  REACTION TO MEASUREMENT CELL             │
          └──────────────────┬───────────────────────┘
  S103                       ▼
          ┌──────────────────────────────────────────┐
          │  IRRADIATE SAMPLE WATER IN MEASUREMENT    │
          │  CELL WITH MEASUREMENT LIGHT              │
          └──────────────────┬───────────────────────┘
  S104                       ▼
          ┌──────────────────────────────────────────┐
          │  DETECT MEASUREMENT LIGHT HAVING PASSED   │
          │            THROUGH SAMPLE WATER           │
          └──────────────────┬───────────────────────┘
  S105                       ▼
          ┌──────────────────────────────────────────┐
          │  CALCULATE ABSORBANCE BASED ON INTENSITY  │
          │            AT EACH WAVELENGTH             │
          └──────────────────┬───────────────────────┘
  S106                       ▼
          ┌──────────────────────────────────────────┐
          │          READ CORRECTION COEFFICIENT      │
          └──────────────────┬───────────────────────┘
  S107                       ▼
          ┌──────────────────────────────────────────┐
          │      CORRECT ABSORBANCE BY CALCULATION    │
          └──────────────────┬───────────────────────┘
                             │
                             ▼
                    ┌──────────────────┐
                    │       END        │
                    └──────────────────┘
```

# FIG. 5

```
                          ╭──────────────╮
                          │    START     │
                          ╰──────┬───────╯
                                 │
   S201                          ▼
   ┌────────────────────────────────────────────────────────┐
   │ CAUSE TURBIDITY STANDARD SOLUTION TO UNDERGO OXIDATION  │
   │                 REACTION IN REACTOR                     │
   └────────────────────────────────────────────────────────┘
   S202                          │
                                 ▼
   ┌────────────────────────────────────────────────────────┐
   │ SUPPLY TURBIDITY STANDARD SOLUTION AFTER OXIDATION      │
   │           REACTION TO MEASUREMENT CELL                  │
   └────────────────────────────────────────────────────────┘
   S203                          │
                                 ▼
   ┌────────────────────────────────────────────────────────┐
   │ IRRADIATE TURBIDITY STANDARD SOLUTION IN MEASUREMENT    │
   │            CELL WITH MEASUREMENT LIGHT                  │
   └────────────────────────────────────────────────────────┘
   S204                          │
                                 ▼
   ┌────────────────────────────────────────────────────────┐
   │ DETECT MEASUREMENT LIGHT HAVING PASSED THROUGH          │
   │             TURBIDITY STANDARD SOLUTION                 │
   └────────────────────────────────────────────────────────┘
   S205                          │
                                 ▼
   ┌────────────────────────────────────────────────────────┐
   │ CALCULATE ABSORBANCE BASED ON INTENSITY AT EACH         │
   │                   WAVELENGTH                            │
   └────────────────────────────────────────────────────────┘
   S206                          │
                                 ▼
   ┌────────────────────────────────────────────────────────┐
   │           UPDATE CORRECTION COEFFICIENT                 │
   └────────────────────────────────────────────────────────┘
                                 │
                                 ▼
                          ╭──────────────╮
                          │     END      │
                          ╰──────────────╯
```

# FIG. 6

## INTERNATIONAL SEARCH REPORT

| International application No. |
|---|
| PCT/JP2021/005250 |

### A. CLASSIFICATION OF SUBJECT MATTER

G01N 21/27(2006.01)i; G01N 21/33(2006.01)i; G01N 31/00(2006.01)i
FI: G01N21/27 F; G01N31/00 F; G01N21/33
According to International Patent Classification (IPC) or to both national classification and IPC

### B. FIELDS SEARCHED

Minimum documentation searched (classification system followed by classification symbols)
G01N1/00-1/44;    G01N21/00-21/01;    G01N21/17-21/61;    G01N21/75-21/83;
G01N31/00; G01N33/00; G01N33/18; C02F1/00-1/72

Documentation searched other than minimum documentation to the extent that such documents are included in the fields searched
| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>Y | JP 02-198341 A (HORIBA, LTD.) 06 August 1990 (1990-08-06) page 1, right column, line 19 to page 2, upper left column, line 11, page 2, upper left column, line 20 to page 2, upper right column, line 8, page 3, upper right column, lines 5-17, page 4, upper right column, line 20 to page 5, lower left column, line 6, fig. 4, 7 | 1-3, 5-6, 8<br>4, 7 |
| Y | WO 2009/050222 A1 (UNILEVER PLC) 23 April 2009 (2009-04-23) specification, page 16, lines 7-25 | 4 |
| Y | WO 2013/121577 A1 (SHIMADZU CORPORATION) 22 August 2013 (2013-08-22) paragraph [0003] | 7 |
| A | JP 2004-093509 A (DKK-TOA CORPORATION) 25 March 2004 (2004-03-25) entire text, fig. 1-19 | 1-8 |
| A | WO 95/01560 A1 (TECATOR AB) 12 January 1995 (1995-01-12) entire text, fig. 1-6 | 1-8 |

☒  Further documents are listed in the continuation of Box C.　　☒　See patent family annex.

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 17 April 2021 (17.04.2021) | 27 April 2021 (27.04.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

| | INTERNATIONAL SEARCH REPORT | International application No. |
| --- | --- | --- |
| | | PCT/JP2021/005250 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | WO 92/16828 A2 (WELSH WATER ENTERPRISES LTD.) 01 October 1992 (1992-10-01) entire text, fig. 1-4b | 1-8 |
| A | JP 64-000461 A (FUJI ELECTRIC CO., LTD.) 05 January 1989 (1989-01-05) entire text, fig. 1-7 | 1-8 |

Form PCT/ISA/210 (continuation of second sheet) (January 2015)

INTERNATIONAL SEARCH REPORT

Information on patent family members

International application No.

PCT/JP2021/005250

| Patent Documents referred in the Report | Publication Date | Patent Family | Publication Date |
|---|---|---|---|
| JP 02-198341 A | 06 Aug. 1990 | (Family: none) | |
| WO 2009/050222 A1 | 23 Apr. 2009 | JP 2011-500044 A paragraph [0064] US 2010/0291630 A1 | |
| WO 2013/121577 A1 | 22 Aug. 2013 | US 2016/0054228 A1 paragraph [0003] | |
| JP 2004-093509 A | 25 Mar. 2004 | (Family: none) | |
| WO 95/01560 A1 | 12 Jan. 1995 | JP 08-512134 A US 5641966 A | |
| WO 92/16828 A2 | 01 Oct. 0992 | JP 06-508431 A US 5420432 A | |
| JP 64-000461 A | 05 Jan. 1989 | (Family: none) | |

Form PCT/ISA/210 (patent family annex) (January 2015)

**REFERENCES CITED IN THE DESCRIPTION**

**Patent documents cited in the description**

- JP 2016080441 A